# EUROPEAN PATENT SPECIFICATION

(11) **EP 1 449 510 B1**
(45) Date of publication and mention of the grant of the patent: **17.12.2008**
(21) Application number: 02788680.3
(22) Date of filing: 28.11.2002
(51) Int. Cl.: A61Q 5/12, A61K 8/898

(54) **HAIR CARE PRODUCTS**
HAARPFLEGEPRODUKTE
PRODUITS DE SOINS CAPILLAIRES

(30) Priority: 28.11.2001 WO PCT/JP01/10409
(43) Date of publication of application: 25.08.2004
(73) Proprietor: Shin-Etsu Chemical Company, Ltd., Tokyo 100-0004 (JP)
(72) Inventor: HIRAI, Motohiko, c/o Shin-Etsu Chemical Co., Ltd., Usui-gun, Gunma 379-0224 (JP); TAKARADA, Mituhiro, c/o Shin-Etsu Chemical Co. Ltd, Usui-gun, Gunma 379-0224 (JP); OMURA, Naoki, c/o Shin-Etsu Chemical Co., Ltd., Tokyo 100-0004 (JP); TACHIBANA, Kiyomi, c/o Shin-Etsu Chemical Co. Ltd., Tokyo 100-0004 (JP)
(74) Representative: Luderschmidt, Schüler & Partner
(86) International application number: PCT/JP2002/012440
(87) International publication number: WO 2003/045341

(56) References cited:
- EP-A- 1 063 344
- EP-A- 1 081 271
- WO-A1-97/35542
- WO-A1-97/35543
- WO-A1-97/35544
- WO-A1-97/35545
- WO-A1-97/35546
- WO-A1-97/35547
- WO-A1-97/35548
- WO-A1-97/35549
- JP-A- 2002 104 934
- US-A- 5 807 956

## Description

### TECHNICAL FIELD

The present invention relates to a hair-care toiletry composition containing an organopolysiloxane having an amino group and a polyoxyalkylene group in the molecule and, more particularly, relates to a hair-care toiletry composition having excellent conditioning effects and usability such as sortability, smoothness, finger pass-through and others of the hair by the content of the aforementioned specific organopolysiloxane and also, by the addition of a polymeric compound for hair fixing as a setting agent, capable of imparting flexibility, lubricity and emollient effect and imparting natural gloss and, while having satisfactory setting retainability, still being excellent in the suppressing effect of falling (flaking) of the film of the polymeric compound for hair fixing and having good stability with time.

### BACKGROUND ART

Along with the increase in the consciousness of hair-caring, in recent years, it is desired for hair-care toiletry compositions such as shampoos, rinses, hair treatments and the like to be excellent in the conditioning effects such as flexibility, smoothness, emollient effect and the like in the hair after use.

While the conditioning agents for shampoos heretofore under use include cationic polymers such as cationated celluloses and the like, silicone compounds and others, the cationic polymers have, though smooth in rinsing off, cumulativeness due to high adsorptivity, exhibiting a defect to cause stickiness and stiffness when used over a long time. The silicone compounds, on the other hand, though excellent in dry feeling and smoothness, are never quite satisfactory in the usability because of lack of emollient effect and occurrence of rigidity and stiffness. In order to enhance the conditioning effects of rinses and hair treatments, there are proposed those with application of an alkyl polyalkyleneglycol ether (see Japanese Patent Kokai 4-230614), those by compounding a silicone derivative or a perfluoropolyether (see Japanese Patent Kokai 4-230615 and Japanese Patent Kokai 4-247014) and others but none is available which is satisfactory with respect to usability.

With an object of hair setting, various polymeric compounds for hair fixing are heretofore used but, though excellent in the setting power, these polymeric compounds for hair fixing have, especially when the compounding amount is increased for increasing the setting power, problems of damaged touch feeling of the hair after drying such as increased stiffness and decreased finger pass-through of the hair and appearance of a white powder which is the film of the hair-fixing polymeric compound falling off (flaking) and a defect of stickiness caused in drying. In order to overcome these defects, proposals are made for a combination of an anionic polymer and a specified polyether-modified silicone (see Japanese Patent Kokai 6-100418), a combination of a branched-chain fatty acid ester and a cationic surface active agent (see Japanese Patent Publication 6-96504) and others but none is fully satisfactory as a hair-care toiletry composition capable of imparting good touch feeling after setting.

The present invention accordingly has an object to solve the defects in these conventional hair-care toiletry compositions. Namely, the object is to provide a hair-care toiletry composition which is excellent in the conditioning effects such as, along with good sortability and finger pass-through of the hair after use, flexibility, smoothness, emollient effect and others and excellent usability without cumulativeness and having good stability with time and, when a hair-fixing polymeric compound is used in combination as a setting agent, capable of imparting flexibility, lubricity and emollient effect and imparting natural gloss to be excellent in the suppressing effect on flaking of the film of the hair-fixing polymeric compound while having good setting retainability and having good stability with time.

### DISCLOSURE OF THE INVENTION

The inventors have discovered that the above-described problems can be solved by a hair-care toiletry composition containing an organopolysiloxane having a specified structure and specified properties in a specified proportion leading to completion of the present invention.

Namely, the present invention is a hair-care toiletry composition characterized by containing a composition consisting of an organopolysiloxane having an amino group and a polyoxyalkylene group in the molecule. By this fact, excellent conditioning effects can be obtained to impart the hair with flexibility, smoothness, emollient effect and others.

A hair-care toiletry composition excellent in the conditioning effects such as sortability, finger pass-through, flexibility, smoothness, emollient effect and others of the hair after use along with light spreadability and refreshing feeling of use as well as excellent feeling of use without cumulativeness can be obtained when, in the hair-care toiletry composition of the present invention, the organopolysiloxane as the composition (A) is combined with (B) a cationic surface active agent or
(A) is combined with (C) an anionic surface active agent and/or an amphoteric active agent.

### BEST MODE FOR PRACTICING THE INVENTION

The present invention is a hair-care toiletry composition characterized by containing 0.01-20% by weight of a composition (A) consisting of a single kind only of or two kinds or more of the organopolysiloxanes having an amino group and a polyoxyalkylene group and represented by the below-given general formulas (2) and (4):

R³R²₂Si-[O-Si(R¹)₂]ₙ-OSiR³R²₂; (2)

R³R²₂Si-[O-Si(R¹)₂]ₙ-OSiR¹₃. (4)

[Commonly to the above-given general formulas (2) and (4), R¹ is a substituted or unsubstituted monovalent hydrocarbon group of 1-20 carbon number, R² is an OX (X being a hydrogen atom or a monovalent hydrocarbon group of the same kind as R¹), R³ is a -R⁴(NR⁵CH₂-CH₂)ₐNR⁵₂ (R⁴ being a divalent hydrocarbon group of 1-8 carbon number and a being 0 or 1),
R⁵ is a hydrogen atom or an organic group containing a polyoxyalkylene group expressed by the formula -CH₂-CH(OH)CH₂O-(C₂H₄O)_{b}-(C₃H₆O)_{c}-Z with the proviso that at least one of the total R⁵s is an organic group containing the aforementioned polyoxyalkylene group (here, b being an integer of 2-30, c being an integer of 0-30 and Z being the aforementioned X or an acyl group). And, n represents 10-200.]

In the following, the present invention is described in detail.

The composition (A) in the present invention is a composition consisting of a single kind only or two kinds or more of the organopolysiloxanes having an amino group and a polyoxyalkylene group and represented by the above-given general formulas (2) and (4) and, in the general formulas (2) and (4) representing the respective organopolysiloxanes constituting the (A), R¹ is a substituted or unsubstituted monovalent hydrocarbon group of 1-20 carbon number and particular examples thereof include alkyl groups such as methyl group, ethyl group, propyl group, butyl group, pentyl group, hexyl group, heptyl group, octyl group, nonyl group, decyl group, dodecyl group, tetradecyl group, hexadecyl group, octadecyl group, eicosyl group and the like; cycloalkyl groups such as cyclopentyl group, cyclohexyl group and the like; aryl groups such as phenyl group, tolyl group and the like; and alkenyl groups such as vinyl group, allyl group and the like as well as halogenoalkyl groups by substitution of chlorine or fluorine for a part or all of the hydrogen atoms in those hydrocarbon groups. Among these groups, in particular, methyl group is industrially preferred. R² is an OX where X is a hydrogen atom or the same monovalent hydrocarbon group as R¹. Particular examples of the monovalent hydrocarbon group as this X include alkyl groups such as methyl group, ethyl group, propyl group, butyl group, pentyl group, hexyl group, heptyl group, octyl group and the like.

The R³ in the above-given general formulas (2) and (4) is represented by -R⁴(NR⁵CH₂CH₂)ₐNR⁵₂ and particular preferable examples of R⁴ include alkylene groups such as methylene group, dimethylene group, trimethylene group, tetramethylene group and the like, of which trimethylene group is preferred. And, a is 0 or 1.

Further, in the organic group containing a polyoxyalkylene group represented by -CH₂-CH(OH)CH₂O-(C₂H₄O)_{b}-(C₃H₆O)_{c}-Z within R⁵, the b in the formula is essentially 2-30 or preferably 2-20 since, when smaller than 2, the hydrophilicity is poor while, when larger than 30, flexibility is insufficient. And, c is essentially 0-30 and preferably 0-10 since, when larger than 30, the hydrophilicity to the hair is insufficient. Further, in order to be excellent in flexibility, smoothness and emollient effect to the hair, the organic group containing the polyoxyalkylene group is essentially in at least 30% by moles or, preferably, in at least 50% by moles.

And, particular examples of the Z in the above-given general formulas (2) and (4) include a hydrogen atom, alkyl groups such as methyl group, ethyl group, propyl group, butyl group and the like, and acyl groups such as acetyl group, benzoyl group and the like and others and methyl group, butyl group and acetyl group are particularly preferred.

The n in the above-given general formulas (2) and (4) is essentially 10-200 since, when smaller than 10, hair is insufficiently imparted with flexibility and lubricity while, when larger than 200, stickiness and stiffness are caused in the hair.

The organopolysiloxanes constituting the composition (A) in the hair-care toiletry composition of the present invention can be easily obtained by the reaction between the organopolysiloxanes represented by the below-given general formulas (6) and (8) and the polyoxyalkylene glycidyl ether represented by the below-given general formula (9):

YR²₂Si-[O-Si(R¹)₂]ₙ-OSiYR²₂; (6)

YR²₂Si-[O-Si(R¹)₂]ₙ-OSiR¹₃ (8)

[R¹ and R² are each the same as given before and Y is a group represented by -R⁴-(NHCH₂CH₂)ₐ-NH₂ (R⁴ and a each being the same as given before) and n is the same as given before.] 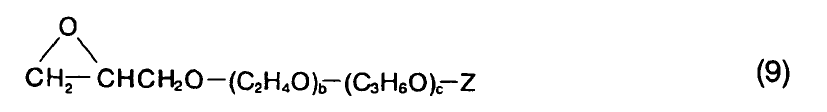 (b, c and Z are each the same as given before.)

Particular examples of the organopolysiloxane represented by the above-given general formulas (6) and (8) include those given below, though not limited thereto.

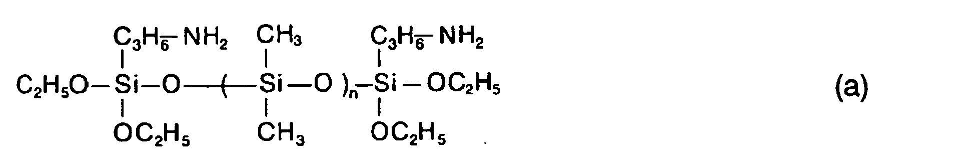

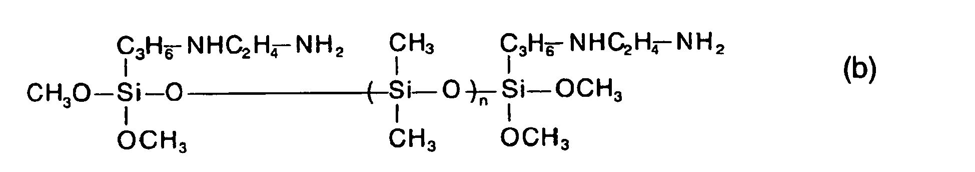

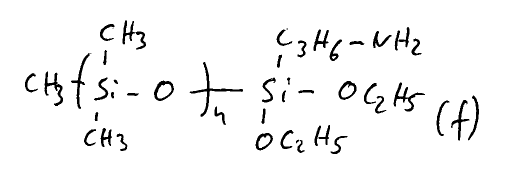

Particular examples of the polyoxyalkylene glycidyl ether represented by the above-given general formula (9) include those given below, though not limited thereto. 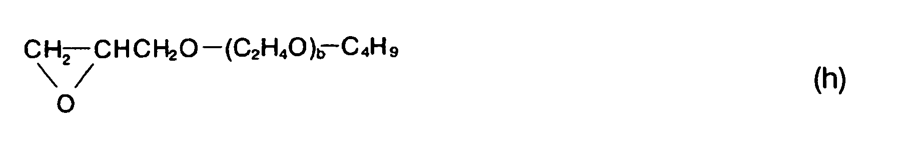 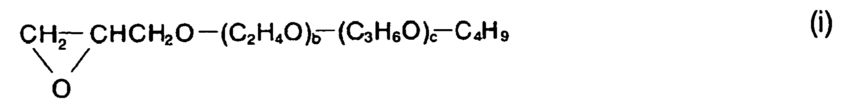 (in the formulas, b and c are each the same as given before)

The organopolysiloxane represented by the above-given general formulas (5)-(8) and the polyoxyaklylene glycidyl ether represented by the general formula (9) react according to the below-given reaction equation 1.

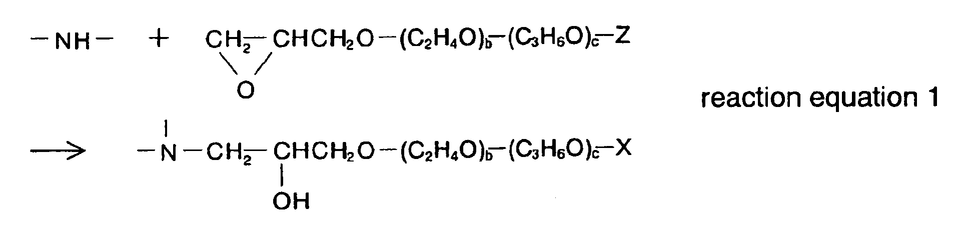

And, in order to improve the flexibility, smoothness and emollient effect to the hair as is mentioned before, the polyoxyalkylene group-containing organic group expressed by -CH₂-CH(OH)CH₂O-(C₂H₄O)_{b}-(C₃H₆O)_{c}-Z within R⁵ in the above-given general formulas (2) and (4) are required essentially in at least 30% by moles or preferably in at least 50% by moles so that the reaction is undertaken with essentially at least 30% by moles or preferably at least 50% by moles of the polyoxyalkylene glycidyl ether represented by the above-given general formula (9) relative to the total NH groups in the organopolysiloxane represented by the above-given general formulas (6) and (8). The reaction between the aforementioned NH group-containing organopolysiloxane and the polyoxyalkylene glyciyl ether should be conducted at 50 °C-100 °C for 1-5 hours either in the absence of any solvents or in a solvent such as lower alcohols, toluene, xylene and the like.

It was found in the hair-care toiletry composition of the present invention that a hair-care toiletry composition having excellent conditioning effects such as good sortability and finger pass-through, flexibility, emollient effect and the like to the hair after use along with light spreadability and refreshing use feeling and excellent usability without cumulativeness could be obtained by combining the organopolysiloxane as the composition (A) with (B) a cationic surface active agent or by combining the (A) with (C) an anionic surface active agent and/or an amphoteric surface active agent. When they were further admixed with (D) a polymeric compound for hair-fixing, furthermore, it was found that the hair was imparted with a use feeling of light spreadability and refreshingness without stickiness and stiffness, touch feeling of wiriness and smoothness, emollient effect and the like but still exhibits suppression of stickiness in drying, falling (flaking) of the film of the fixing polymeric compound and further good setting retainability.

The compounding amount of the organopolysiloxane as the composition (A) in the hair-care toiletry composition of the present invention is preferably 0.01-20% by weight or, more preferably, 0.05-10% by weight from the standpoint of exhibition of the effects and usability. When the compounding amount is smaller than 0.01% by weight, no sufficient effects can be obtained while an excess over 20% by weight is undesirable in respect of usability. As the (A), the organopolysiloxanes represented by the general formulas (2) and (4) can be used either as a single kind alone or as a combination of any two kinds or more and the compounding proportion of these four types of organopolysiloxanes is not particularly limitative. This is because, derived from the preparation method of the amino group-containing organopolysiloxanes as the base material of the organopolysiloxanes represented by the general formulas (2) and (4), it is sometimes the case that a mixture of the general formulas (2) and (4) is obtained.

As the cationic surface active agent as the component (B) in the hair-care toiletry composition of the present invention, any one among those used in conventional toiletry compositions can be used. There can be named, for example, alkyltrimethylammonium chlorides, stearyltrimethylammonium chloride, lauryltrimethylammonium chloride, cetyltrimethylammonium chloride, tallow alkyltrimethylammonium chloride, behenyltrimethylammonium chloride, stearyltrimethylammonium bromide, behenyltrimethylammonium bromide, distearyldimethylammonium chloride, dilauryldimethylammonium chloride, dioctyldimethylammonium chloride, polyoxyethylene oleylmethylammonium (2E.O.) chloride, benzalkonium chloride, stearyl dimethyl benzylammonium chloride, lanolin-derived quaternary ammonium salts, stearic acid diethylaminoethylamide, stearic acid dimethylaminopropylamide, propyldimethylhydroxypropylammonium behenic acid amide and the like.

The compounding amount of the component (B) in the hair-care toiletry composition of the present invention is preferably 0.01-20% by weight or, more preferably, 0.05-10% by weight in order to impart the hair with good touch feeling such as smoothness and wettishness. When the compounding amount is smaller than 0.01% by weight, the conditioning effects to the hair would be insufficient while an excess over 20% by weight results in an undesirable touch feeling such as stickiness, oiliness and the like.

As the anionic surface active agent and/or the amphoteric surface active agent as the component (C) used in the present invention, any one of those used in conventional toiletry compositions can be used. There can be named, for example, as an anionic surface active agent, saturated or unsaturated fatty acid soaps, polyoxyethylene sulfate ester salts, α -acylsulfonate ester salts, alkylsulfonate salts, alkylarylsulfonate salts, α -olefinsulfonate salts, alkylbenzenesulfonate salts, alkylnaphthalenesulfonate salts, alkanesulfonate salts, alkyl- or alkenylsulfate salts, alkylamide sulfate salts, alkyl- or alkenylphosphate salts, alkylamidephosphate salts, alkyloylalkyl taurine salts, N-acylaminoacid salts, sulfosuccinic acid salts, alkylether carboxylic acid salts, amidoether carboxylic acid salts, α -sulfofattyacid ester salts and the like.

As the amphoteric surface active agent, there can be named those of the carboxybetaine type, amidobetaine type, sulfobetaine type, hydroxysulfobetaine type, amidosulfobetaine type, phosphobetaine type, aminocarboxylic acid salt type, imidazoline derivative type, amidoamine type and the like. When both of the anionic surface active agent and the amphoteric surface active agent are used in combination as the component (C), meanwhile, the compounding proportion of both can be freely selected.

The compounding amount of the component (C) in the hair-care toiletry composition of the present invention is preferably 0.01-20% by weight or, more preferably, 0.05-10% by weight in order to impart the hair with good touch feeling such as smoothness and wettishness. When the compounding amount is smaller than 0.01 % by weight, the conditioning effects to the hair would be insufficient while an excess over 20% by weight would result in undesirable touch feeling such as stickiness and oiliness.

The polymeric compound for hair fixing as the component (D) in the hair-care toiletry composition of the present invention includes those polymeric compounds of the amphoteric, anionic, cationic and non-ionic types and there can be named polyvinylpyrrolidones, vinylpyrrolidone/vinyl acetate copolymers, vinylpyrrolidone/vinyl acetate/vinyl propionate ternary copolymers, vinyl pyrrolidone/alkylamino(meth)acrylate (quaternary chloro) copolymers, vinyl pyrrolidone/alkyl (meth)acrylate/(meth)acrylic acid copolymers, vinyl pyrrolidone/alkylamino(meth)acrylate/vinyl caprolactam copolymers, vinyl pyrrolidone/methyl vinyl imidazolium chloride and the like as the vinyl pyrrolidone-based polymeric compound; methyl vinyl ether/maleic anhydride alkyl half ester copolymers and the like as the acidic vinyl ether-based polymeric compound; vinyl acetate/crotonic acid copolymers, vinyl acetate/crotonic acid/vinyl neodecanoate copolymers, and the like as the acidic polyvinyl acetate-based polymer; (meth)acrylic acid/alkyl (meth)acrylate copolymers, (meth)acrylic acid/alkyl (meth)acrylate/alkyl acrylamide copolymers and the like as the acidic acrylic polymeric compound; N-methacryloylethyl-N,N-dimethylammonium α -N-methylcarboxybetaine/alkyl (meth)acrylate copolymers, hydroxypropyl (meth)-acrylate/butyl aminoethyl methacrylate/acrylic acid octylamide copolymerds and the like as the amphoteric acrylic-based polymeric compound. In addition, natural polymeric compounds such as cellulose or derivatives thereof, keratin and collagen or derivatives thereof and the like can also be used satisfactorily.

The polymeric compound for hair fixing as the component (D) in the hair-care toiletry composition of the present invention is effective in imparting the hair with a refreshing feeling and the compounding amount of the component (D) in the hair-care toiletry composition is preferably 0.01-20% by weight or, more preferably, 0.05-10% by weight. When the compounding amount is smaller than 0.01 % by weight, the effect of setting and the like would be insufficient while an excess over 20% by weight would undesirably result in an increase of stiffness, dry and loose feeling and the like of the hair.

When the amphoteric or anionic polymeric compound for hair fixing is used, meanwhile, it is optional according to need that a part or all of the functional groups thereof are neutralized with an organic amine compound such as 2-amino-2-methyl-1-propanol, triethanolamine and the like or an alkali agent such as potassium hydroxide and the like. And, these polymeric compounds for hair fixing (D) can be used, according to need, either singly or as a mixture of two kinds or more.

In addition to the above-described components, it is possible that the hair-care toiletry composition of the present invention is compounded with other ingredients conventionally compounded in hair-care toiletry compositions including viscosity-controlling agents, film-forming agents, hair-nature improvers, pH controlling agents, detergents, emulsifiers, emulsification promotors, propellants and the like each in such a range as not to decrease the advantages of the invention according to the object.

As the viscosity-controlling agent, water-soluble polymers such as methyl cellulose, carboxymethyl cellulose, hydroxyethyl cellulose, xanthan gum and the like, non-ionic surface active agents such as fatty acid alkylolamides and the like and others can be used and, as the film-forming agent, cationic polymers such as cationated cellulose, cationated starch, cationated guar gum, vinyl pyrrolidone/N,N-dimethylaminoethyl methacrylic acid copolymeric quaternarized compounds, diallyl quaternary ammonium salt polymers and the like; non-ionic polymers such as polyvinylpyrrolidone, polyvinylpyrrolidone/vinyl acetate copolymers, polyvinyl alcohols and the like; anionic polymers such as methyl vinyl ether/maleic acid half ester copolymers, acrylic resin alkanolamine solutions and the like; amphoteric copolymers such as N-methacryloyloxyethylene N,N-dimethylammonium- α -N-methyl-carboxybetaine/methacrylic acid alkyl ester copolymers and the like; and others can be used.

And, there can be used, as the hair-nature improvers, silicone derivatives such as low-viscosity silicones, high-polymeric silicones, cyclic silicones, polyether-modified silicones, amino-modified silicones, cationic silicones and the like, as the pH controlling agent, acids such as citric acid, lactic acid and the like or salts thereof, as the emulsifying agent, non-ionic surface active agents such as polyoxyalkylene-addition type surface active agents and the like, as the emulsification promotors, higher alcohols or glycerin fatty acid esters and the like, and, as the propellant, liquefied petroleum gas, nitrogen gas, carbon dioxide gas, dimethyl ether and the like. It is further optional to compound, in addition thereto, other ingredients conventionally compounded in toiletry compositions including oleaginous ingredients such as ester compounds having a straight chain or branched chain, hydrocarbons, fats and oils and the like, water-base ingredients such as polyhydric alcohols, lower alcohols and the like, perfumes, antiseptic agents, ultraviolet-absorbers, antioxidants, antibacterial or bactericidal agents, moisturizing agents, salts, chelating agents, refreshing agents, anti-inflammatory agents, beauty-skin ingredients (whitening agents, cell-invigorators, rough skin improvers, blood circulation promotors, skin astringents, antiseborrheic agents and the like) vitamins, aminoacids, nucleic acids, hormones, clathrate compounds and others.

Depending on concurrent use with other ingredients or working types of the container, the hair-care toiletry composition of the present invention can be rendered to practice in a variety of forms including liquid form, milky form, creamy form, solid form, pasty form, gelled form, mousse form, misty form and others without being limited to particular preparation forms.

### (Examples)

In the following, the present invention is described in particulars by making reference to Examples although the present invention is never limited thereto. Meanwhile, those described in the Examples merely in % and the numerical figures for the respective ingredients in Tables 1-3 all refer to % by weight.

### (Preparation Example 1)

By introducing, into a separable flask of 50 ml capacity equipped with a thermometer, stirrer equipment, reflux condenser and nitrogen gas inlet tube, 100 g of an amino group-containing organopolysiloxane (molecular weight 3328) expressed by the below-given chemical formula (aa), 39 g of a polyoxyalkylene glycidyl ether (molecular weight 333) expressed by the below-given chemical formula (bb) (100% by moles relative to the total NH groups in the aminoalkyl group-containing organopolysiloxane) and 13.9 g of isopropyl alcohol, a reaction was carried out at 80 °C for 4 hours under hermetic sealing after introduction of nitrogen gas. After completion of the reaction, low boiling point fractions were removed at 80 °C for 1 hour under a reduced pressure of 10 mmHg to obtain 136 g of an oily material of 2.4% volatile matter having a viscosity of 1240 mPa.s and an amine equivalent of 2410 g/mole [a value measured with an automatic titration apparatus by using a 0.01 N aqueous hydrochloric acid solution for about 1.5 g of this product dissolved in about 50 ml of a 1:1 mixed solvent of toluene and isopropyl alcohol. Even after consumption of all of the epoxy groups indicated in the below-given chemical formula (cc), detection as the amine equivalent can be made by this measuring method.]

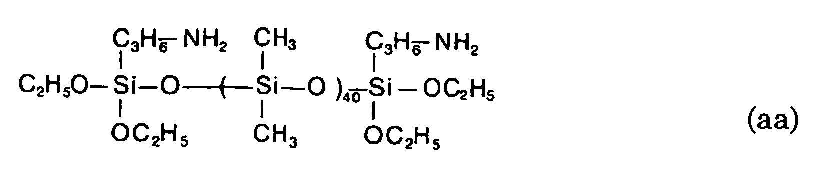

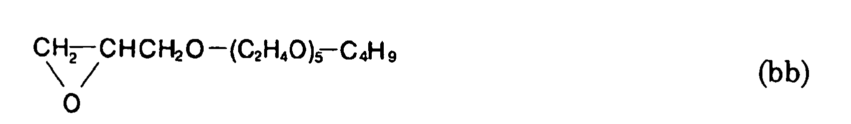

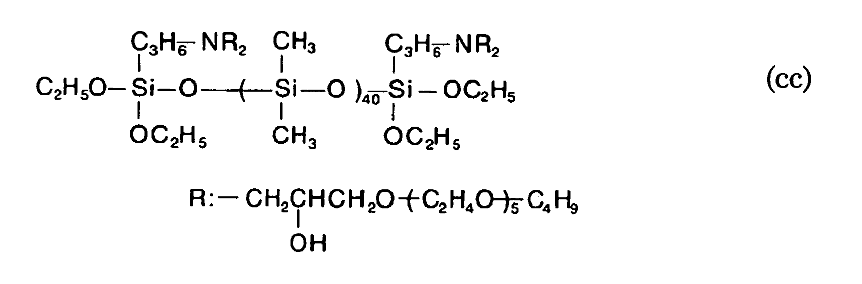

### (Preparation Example 2)

By using 100 g of the aminoalkyl group-containing organopolysiloxane expressed by the chemical formula (aa) as used in Preparation Example 1 and 25.4 g of the polyoxyalkylene glycidyl ether expressed by the chemical formula (bb) (65% by moles relative to the total NH in the aminoalkyl group-containing organopolysiloxane), a reaction was carried out to obtain 119 g of an oily compound of 2.0% of volatile matter having a viscosity of 950 mPa.s and an amine equivalent of 2050 g/mole as expressed by the below-given chemical formula (dd).

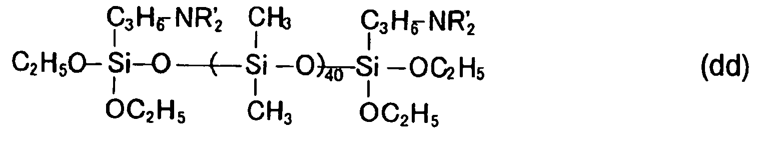

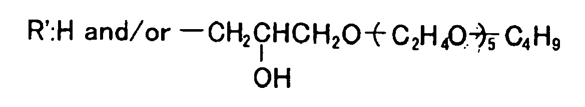

### (Preparation Example 3; not within the scope of claim 1)

By introducing 100 g of an aminoalkyl group-containing organopolysiloxane (molecular weight 3326) expressed by the below-given chemical formula (ee), 49.8 g of the polyoxyalkylene glycidyl ether expressed by the chemical formula (bb) (100% by moles relative to the total NH groups in the aminoalkyl group-containing organopolysiloxane) and 7.5 g of isopropyl alcohol, a reaction was carried out at 80 C for 4 hours under hermetic sealing after introduction of nitrogen gas. After completion of the reaction, low boiling point fractions were removed at 80 °C for 1 hour under a reduced pressure of 10 mmHg to obtain 143 g of an oily material of 1.5% volatile matter having a viscosity of 746 mPa.s and an amine equivalent of 3020 g/mole as expressed by the below-given chemical formula (ff).

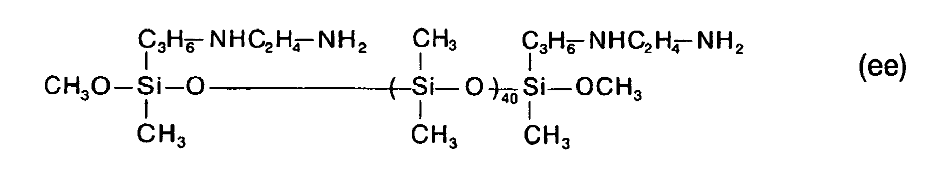

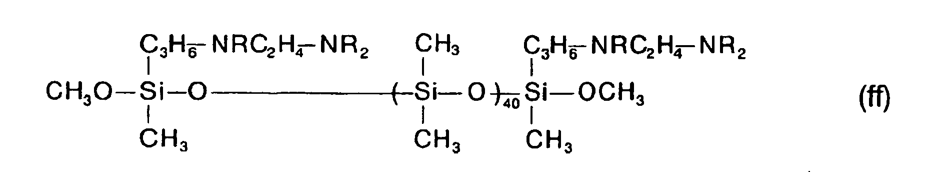

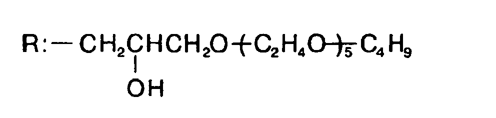

### (Preparation Example 4; not within the scope of claim 1)

By introducing 100 g of an aminoalkyl group-containing organopolysiloxane (molecular weight 1997) expressed by the below-given chemical formula (gg), 57.5 g of a polyoxyalkylene glycidyl ether expressed by the chemical formula (ii) (100% by moles relative to the total NH groups in the aminoalkyl group-containing organopolysiloxane) and 15.8 g of isopropyl alcohol, a reaction was carried out at 80 °C for 4 hours under hermetic sealing after introduction of nitrogen gas. After completion of the reaction, low boiling point fractions were removed at 80 °C for 1 hour under a reduced pressure of 10 mmHg to obtain 156 g of an oily material of 1.3% volatile matter having a viscosity of 350 mPa.s and an amine equivalent of 5560 g/mole as expressed by the below-given chemical formula (hh).

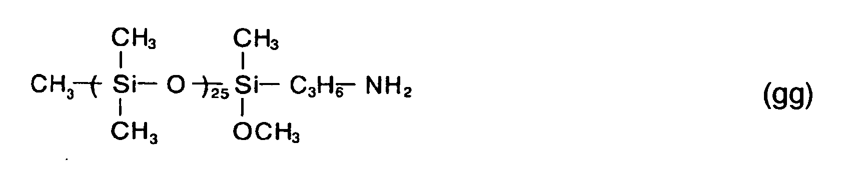

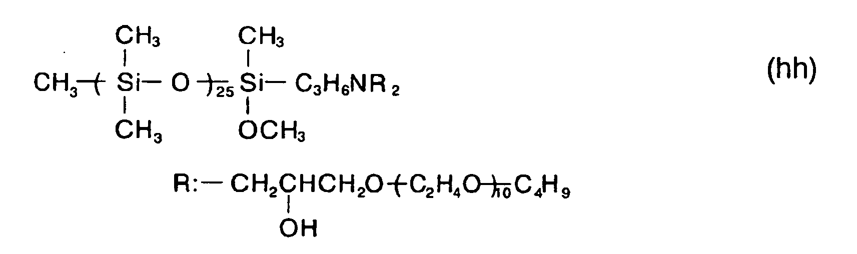

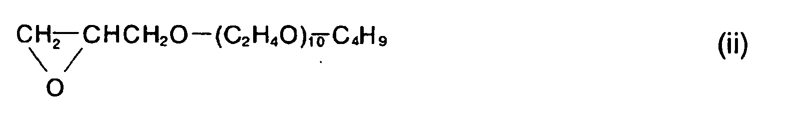

### (Examples 1-3 and Comparative Examples 1-3): Hair conditioners

Hair conditioners of the respective formulations indicated in Table 1 were prepared and evaluation was made by the method described below for the use feeling and usability thereof to give the results shown in Table 1.

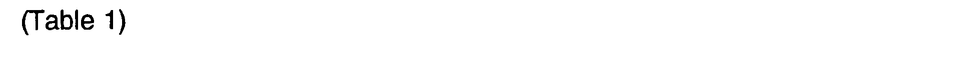

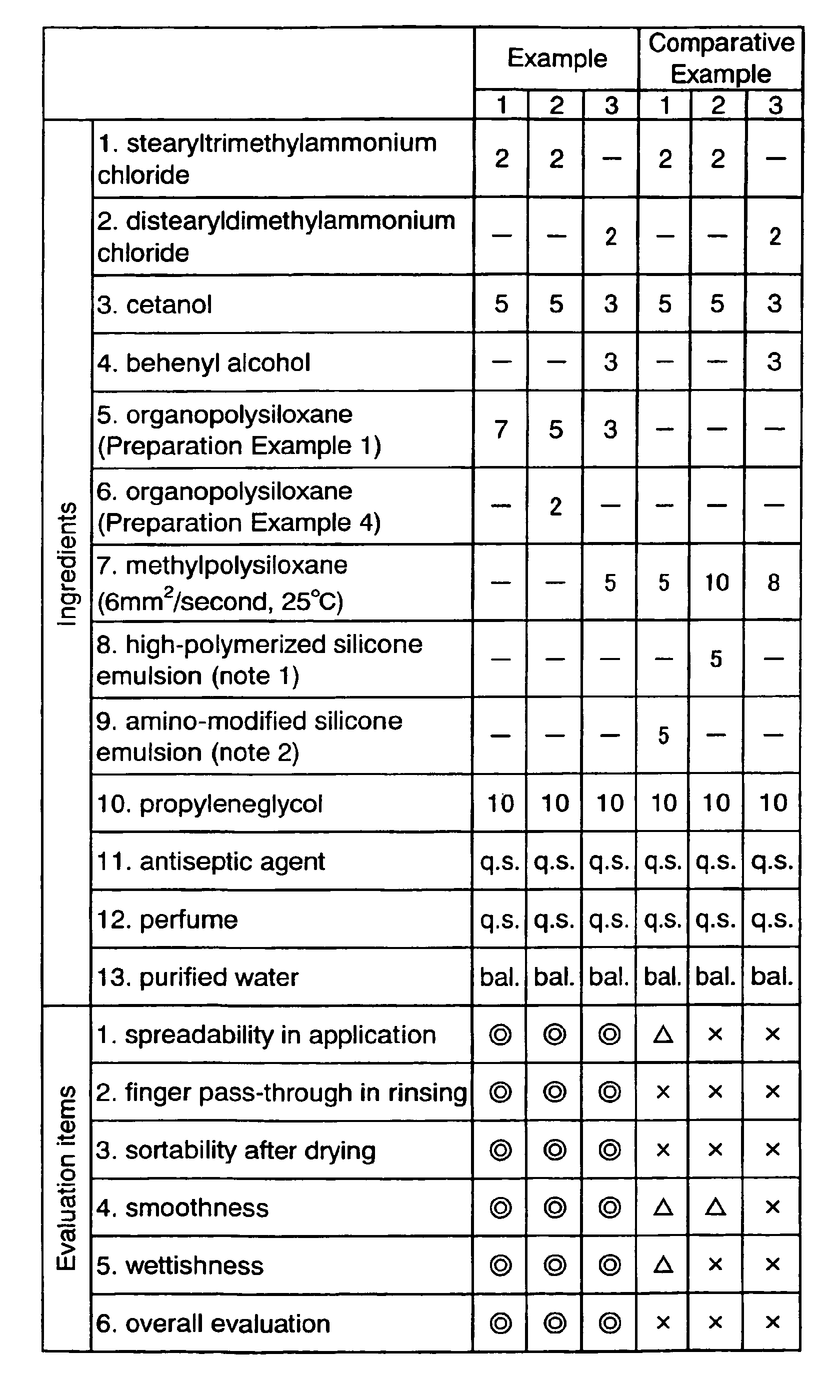

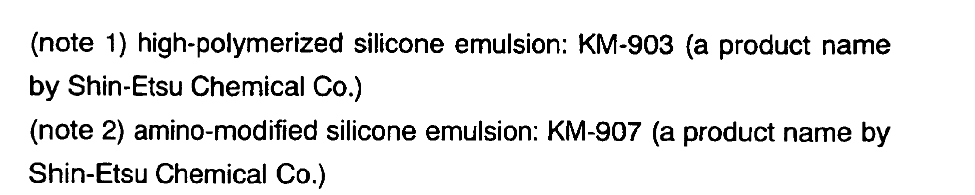

### (Preparation method of hair conditioners)

Step A: Ingredients 1-2, 10-11 and 13 are uniformly mixed and dissolved by heating.
Step B: Ingredients 3-9 are uniformly mixed and dissolved by heating.
Step C: The mixture of step B is added to the mixture of step A and emulsified followed by cooling with addition of the ingredient 12 to obtain the hair conditioner.

### (Evaluation method)

Use tests were conducted by 20 female expert panel members in which shampooing with a commercial shampoo product was followed by application of the hair conditioner for the spreadability in application, finger pass-through in rinsing followed by drying of the hair, and sortability, smoothness and wettishness of the hair after drying to make evaluations according to the following criteria and rating by way of the average points thereof.

### (Evaluation)

### [Criteria of evaluation]

5 points: excellent
4 points: good
3 points: fair
2 points: poor
1 point: unacceptable

### [Rating]

Ⓞ: 4.5 or higher average points
O: 3.5 or higher but lower than 4.5 average points
Δ: 2.5 or higher but lower than 3.5 average points
×: lower than 2.5 average points

As was clear from the results of Table 1, the hair conditioners of Examples 1-3 formulated with the organopolysiloxanes of the present invention, as compared with those of Comparative Examples 1-3, exhibited very excellent effects in respects of spreadability in application, finger pass-through in rinsing and sortability, smoothness and wettishness of the hair after drying so that they were found synthetically very excellent.

### (Examples 4-5 and Comparative Examples 4-5): Hair creams

Hair creams of the respective formulations indicated in Table 2 were prepared and evaluation was made for the use feeling and usability thereof to give the results shown in Table 2.

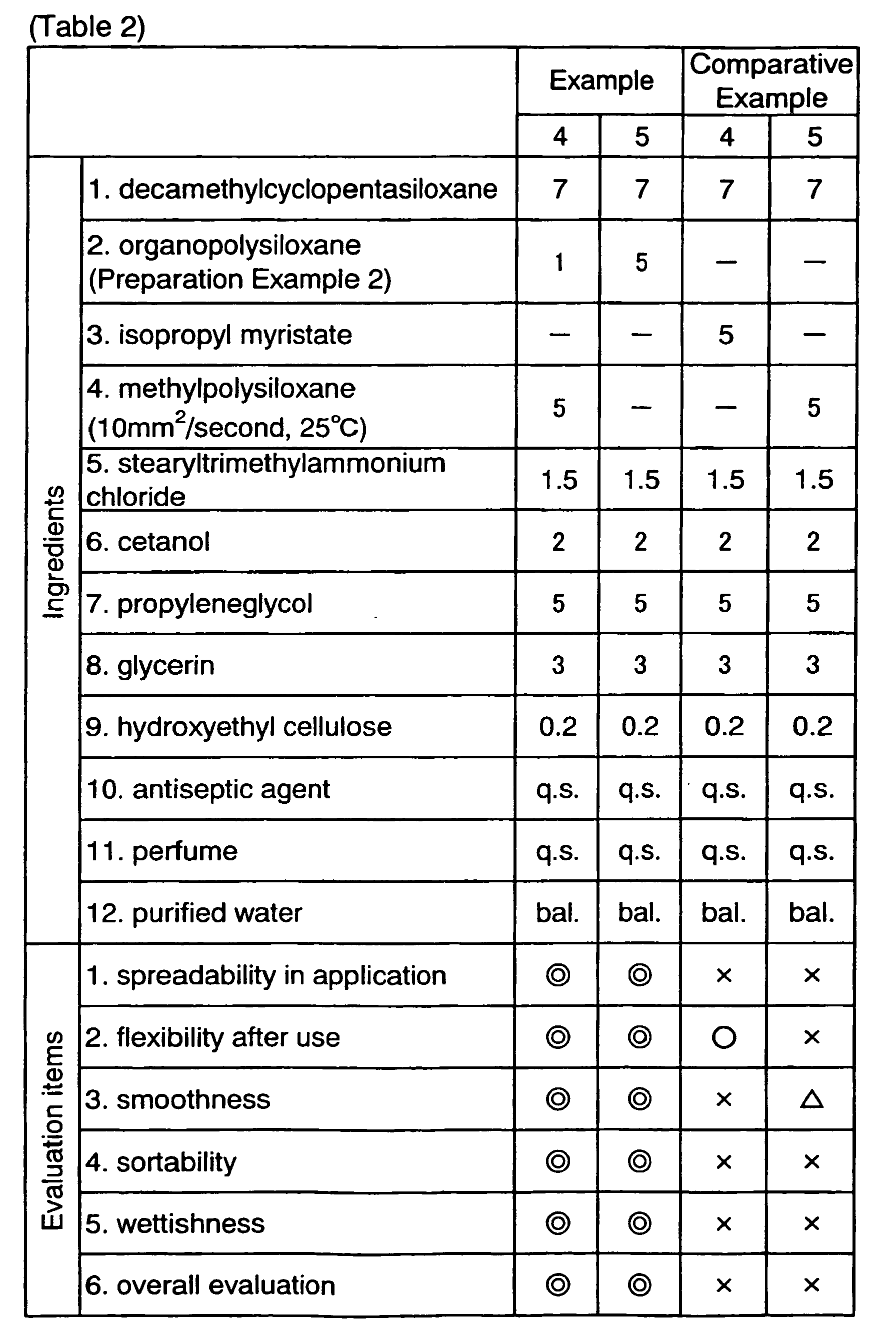

### (Preparation method)

Step A: Ingredients 1-4 and 6 are uniformly mixed and dissolved by heating.
Step B: Ingredients 5, 7-10 and 12 are uniformly mixed and dissolved by heating.
Step C: The mixture of Step A is added to the mixture obtained in Step B and emulsified followed by cooling with addition of the ingredient 11 to obtain the hair cream.

### (Evaluation)

Evaluation was made according to the above-given criteria for the spreadability in application and flexibility, smoothness, sortability and wettishness of the hair after use and rating was made by way of the average points thereof.

As is clear from the results of Table 2, the hair creams of Examples 4 and 5 compounded with the organopolysiloxanes of the present invention exhibited, as compared with those of Comparative Examples 4 and 5, very excellent effects in respects of spreadability in application and flexibility, smoothness, sortability and wettishness of the hair after use so that they were found synthetically very excellent.

### (Examples 6-9 and Comparative Examples 6-8): Styling mousses

Styling mousses of the respective formulations indicated in Table 3 were prepared and evaluation was made for the use feeling and usability thereof to give the results shown in Table 3.

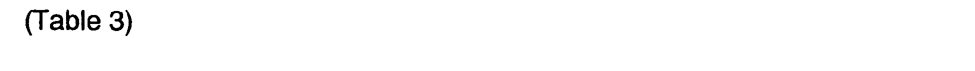

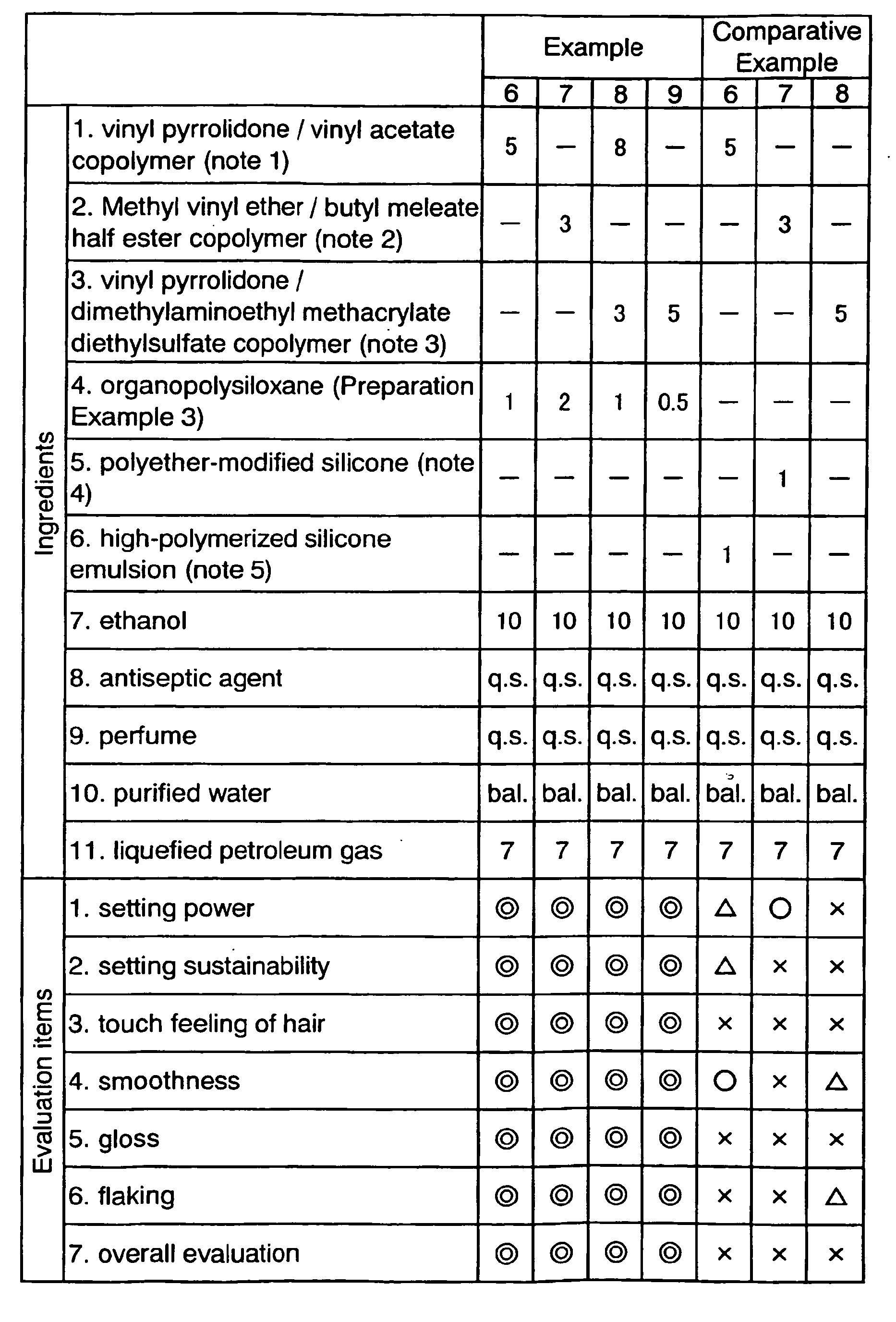

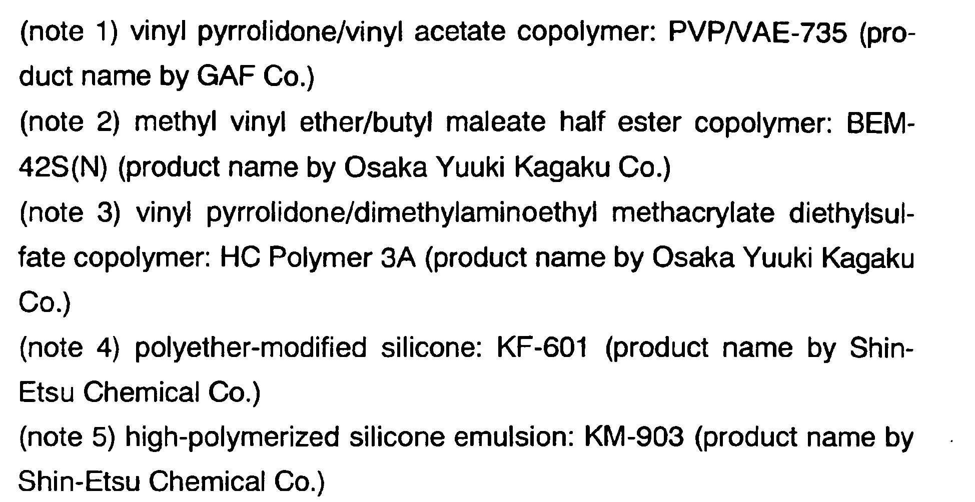

### (Preparation method)

Step A: Ingredients 1-11 are uniformly mixed.
Step B: An aerosol can is impregnated with the mixture obtained in Step A and the ingredient 12 to obtain the styling mousse.

### (Evaluation)

Commercial shampoo and rinse of conventional types were applied to wigs of human hair followed by drying. Thereafter, each a 5 g portion of the styling mousses of Examples 6-9 and Comparative Examples 6-8 was applied to the wigs followed by drying, of which evaluation was made according to the aforementioned criteria for the conditions of degree of setting power, setting sustainability, touch feeling of the hair such as stickiness and stiffness, smoothness and gloss as well as for absence of flaking after five times of combing (letting down of hair with a comb) of the wigs to give ratings with the average points thereof.

As was clear from the results of Table 3, it was understood that the styling mousses of Examples 6-9 exhibited, as compared with those of Comparative Examples 6-8, very excellent effects in respects of the conditions of degree of setting power, setting sustainability, touch feeling of the hair such as stickiness and stiffness, smoothness, gloss and flaking to be synthetically very excellent.

### (Example 10): Hair cream for hair dressing

| | (Name of ingredient) | (%) |
|---|---|---|
| 1. | Organopolysiloxane (Preparation Example 5) | 5.0 |
| 2. | Stearic acid | 0.5 |
| 3. | Squalane | 5.0 |
| 4. | Liquid paraffin | 20.0 |
| 5. | Polyoxyethylene cetyl ether (20 E.O.) | 1.5 |
| 6. | Polyoxyethylene cetyl ether (2 E.O.) | 2.5 |
| 7. | Sorbitan monostearate | 1.5 |
| 8. | Glyceryl monostearate (self-emulsification type) | 0.5 |
| 9. | Triethanolamine | 1.0 |
| 10. | Polyvinyl pyrrolidone | 10.0 |
| 11. | Polyacrylic acid amide mixed solution (note 1) | 2.0 |
| 12. | Propyleneglycol | 5.0 |
| 13. | Antiseptic agent | q.s. |
| 14. | Perfume | q.s. |
| 15. | Purified water | balance |

| | | |
|---|---|---|
| (note 1) Polyacrylic acid amide mixed solution: Sepigel 305 (product by Sepic Co.) | | |

### (Preparation method)

Step A: Ingredients 1-8 are uniformly mixed and dissolved by warming.
Step B: Ingredients 9-13 and 15 are uniformly mixed and dissolved by warming.
Step C: The mixture obtained in Step B is added to the mixture obtained in Step A and emulsified followed by cooling and addition of the ingredient 14 to obtain the hair cream for hair dressing.

The hair cream of the present invention for hair dressing was free from feeling of oiliness, exhibited an adequate effect of hair dressing without flaking and exhibited very excellent effect in respect of flexibility, wettishness and gloss.

### (Example 11): Shampoo

| | (Name of ingredient) | (%) |
|---|---|---|
| 1. | Organopolysiloxane (Preparation Example 3) | 1.0 |
| 2. | Sodium coconut oil fatty acid methyltaurine | 20.0 |
| 3. | Coconut oil fatty acid amide propylbetaine | 5.0 |
| 4. | Diethanolamide laurate | 3.0 |
| 5. | Antiseptic agent | q.s. |
| 6. | Perfume | q.s. |
| 7. | Purified water | balance |

### (Preparation method)

### Step A: Ingredients 1-7 are uniformly mixed.

The shampoo of the present invention gave, after shampooing, sortability, smoothness and wettishness and exhibited a very excellent effect in respect of gloss.

### INDUSTRIAL APPLICABILITY

The hair-care toiletry compositions compounded with an organopolysiloxane according to the present invention exhibit, along with light spreadability and refreshing use feeling, excellent conditioning effects on the hair after use such as sortability, good finger pass-through, flexibility, smoothness, emollient effect and the like as well as excellent usability without cumulativeness and good stability with time.

Further, it is a very excellent hair-care toiletry composition because, when a polymeric compound for hair fixing is used in combination as a setting agent, it imparts flexibility, smoothness and emollient effect, gives natural gloss and has good setting retainability but still is excellent suppressing effect against falling (flaking) of the film of the polymeric compound for fixing and has good stability with time.

## Claims

1. A hair-care toiletry composition **characterized by** containing 0.01-20% by weight of a composition (A) consisting of a single kind or two kinds or more of the organopolysiloxanes having an amino group and a polyoxyalkylene group as represented by the general formulas (2) and (4) given below:
R³R²₂Si-[O-Si(R¹)₂]ₙ-OSiR³R²₂; (2)
R³R²₂Si-[O-Si(R¹)₂]ₙ-OSiR¹₃. (4)
[common to the above-given general formulas (2) and (4), R¹ is a substituted or unsubstituted monovalent hydrocarbon group of 1-20 carbon number, R² is an OX (X being a hydrogen atom or a monovalent hydrocarbon group of the same kind as R¹), R³ is -R⁴(NR⁵CH₂CH₂)ₐNR⁵₂ (R⁴ being a divalent hydrocarbon group of 1-8 carbon number and a being 0 or 1), R⁵ is a hydrogen atom or a polyoxyalkylene group-containing organic group expressed by the formula -CH₂-CH(OH)CH₂O-(C₂H₄O)_{b}-(C₃H₆O)_{c}-Z with the proviso that at least one of the total R⁵ is the aforementioned polyoxyalkylene group-containing organic group (here, b being an integer of 2-30, c being an integer of 0-30, Z being the aforementioned X or an acyl group) and n represents 10-200]

2. The hair-care toiletry composition described in Claim 1 wherein R² is an OCH₃ group or an OC₂H₅ group.

3. The hair-care toiletry composition described in Claim 1 wherein the -CH₂-CH(OH)CH₂O-(C₂H₄O)_{b}-(C₃H₆O)_{c}-Z groups occupy at least 30% by moles of total R⁵s.

4. The hair-care toiletry composition described in Claim 1 wherein b is 2-20.

5. The hair-care toiletry composition described in Claim 1 wherein c is 0-10.

6. The hair-care toiletry composition described in either one of Claims 1-5 **characterized by** containing a cationic surface active agent as the component (B).

7. The hair-care toiletry composition described in either one of Claims 1-6 **characterized by** containing an anionic surface active agent and/or an amphoteric surface active agent as the component (C).

8. The hair-care toiletry composition described in either one of Claims 1-7 **characterized by** containing a polymeric compound for hair fixing as the component (D).

## Patentansprüche

1. Haarpflegetoilettenartikel-Zusammensetzung, **dadurch gekennzeichnet, dass** sie 0,01 bis 20 Gew.-% einer Zusammensetzung (A) enthält, bestehend aus einer einzelnen Art oder zwei Arten oder mehr der Organopolysiloxane mit einer Aminogruppe und einer Polyoxyalkylengruppe, wie sie durch die allgemeinen Formeln (2) und (4) wie unten angegeben dargestellt werden:
R³R²₂Si-[O-Si(R¹)₂]ₙ-OSiR³R²₂, (2)
R³R²₂Si-[O-Si(R¹)₂]ₙ-OSiR¹₃. (4)
[Üblicherweise ist in den oben angegebenen allgemeinen Formeln (2) und (4) R¹ eine substituierte oder unsubstituierte einwertige Kohlenwasserstoffgruppe mit einer Kohlenstoffanzahl von 1 bis 20, R² ist ein OX (wobei X ein Wasserstoffatom oder eine einwertige Kohlenwasserstoffgruppe der gleichen Art wie R¹ ist), R³ ist -R⁴(NR⁵CH₂CH₂)ₐNR⁵₂ (wobei R⁴ eine zweiwertige Kohlenwasserstoffgruppe mit einer Kohlenstoffanzahl von 1 bis 8 und a 0 oder 1 ist), R⁵ ist ein Wasserstoffatom oder eine Polyoxyalkylengruppe enthaltende organische Gruppe, ausgedrückt durch die Formel -CH₂-CH(OH)CH₂O-(C₂H₄O)_{b}-(C₃H₆O)_{c}-Z, unter der Voraussetzung, dass zumindest eines von allen R⁵ die zuvor genannte Polyoxyalkylengruppe enthaltende organische Gruppe ist (hierbei ist b eine ganze Zahl von 2 bis 30, c eine ganze Zahl von 0 bis 30, Z das zuvor erwähnte X oder eine Acylgruppe) und n 10 bis 200 darstellt.]

2. Haarpflegetoilettenartikel-Zusammensetzung wie in Anspruch 1 beschrieben, wobei R² eine OCH₃-Gruppe oder eine OC₂H₅-Gruppe ist.

3. Haarpflegetoilettenartikel-Zusammensetzung wie in Anspruch 1 beschrieben, wobei die -CH₂-CH(OH)CH₂O-(C₂H₄O)_{b}-(C₃H₆O)_{c}-Z-Gruppen zumindest 30 mol.-% der gesamten R⁵ besetzen.

4. Haarpflegetoilettenartikel-Zusammensetzung wie in Anspruch 1 beschrieben, wobei b 2 bis 20 ist.

5. Haarpflegetoilettenartikel-Zusammensetzung wie in Anspruch 1 beschrieben, wobei c 0 bis 10 ist.

6. Haarpflegetoilettenartikel-Zusammensetzung wie in einem der Ansprüche 1 bis 5 beschrieben, **dadurch gekennzeichnet, dass** sie ein kationisches oberflächenaktives Mittel als Komponente (B) enthält.

7. Haarpflegetoilettenartikel-Zusammensetzung wie in einem der Ansprüche 1 bis 6 beschrieben, **dadurch gekennzeichnet, dass** sie ein anionisches oberflächenaktives Mittel und/oder ein amphoterisches oberflächenaktives Mittel als Komponente (C) enthält.

8. Haarpflegetoilettenartikel-Zusammensetzung wie in irgendeinem der Ansprüche 1 bis 7 beschrieben, **dadurch gekennzeichnet, dass** sie eine polymere Verbindung für die Haarfixierung als Komponente (D) enthält.

## Revendications

1. Composition d'article de soins capillaires **caractérisée par le fait qu'**elle contient 0,01 à 20 % en poids d'une composition (A) constituée d'un seul type ou de deux types ou plus des organopolysiloxanes ayant un groupe amino et un groupe poly(oxyalkylène) comme représenté par les formules générales (2) et (4) données ci-dessous :
R³R²₂Si-[O-Si(R¹)₂]ₙ-OSiR³R²₂; (2)
R³R²₂Si-[O-Si(R¹)₂]ₙ-OSiR¹₃; (4)
[commun aux formules générales (2) et (4) données ci-dessus, R¹ est un groupe hydrocarbure monovalent substitué ou non substitué ayant un nombre d'atomes de carbone de 1 à 20, R² est un groupe OX (X étant un atome d'hydrogène ou un groupe hydrocarbure monovalent du même type que R¹), R³ est -R⁴(NR⁵CH₂CH₂)ₐNR⁵₂ (R⁴ étant un groupe hydrocarbure divalent ayant un nombre d'atomes de carbone de 1 à 8 et a valant 0 ou 1), R⁵ est un atome d'hydrogène ou un groupe organique contenant un groupe poly(oxyalkylène) exprimé par la formule -CH₂-CH(OH)CH₂O-(C₂H₄O)_{b}-(C₃H₆O)_{c}-Z à condition qu'au moins l'un des R⁵ totaux soit le groupe organique contenant un groupe poly(oxyalkylène) mentionné ci-dessus (ici, b étant un entier de 2 à 30, c étant un entier de 0 à 30, Z étant le X mentionné ci-dessus ou un groupe acyle) et n représente 10 à 200].

2. Composition d'article de soins capillaires décrite dans la revendication 1, dans laquelle R² est un groupe OCH₃ ou un groupe OC₂H₅.

3. Composition d'article de soins capillaires décrite dans la revendication 1, dans laquelle les groupes -CH₂-CH(OH)CH₂O-(C₂H₄O)_{b}-(C₃H₆O)_{c}-Z occupent au moins 30 % en moles des R⁵ totaux.

4. Composition d'article de soins capillaires décrite dans la revendication 1, dans laquelle b vaut 2 à 20

5. Composition d'article de soins capillaires décrite dans la revendication 1, dans laquelle c vaut 0 à 10.

6. Composition d'article de soins capillaires décrite dans l'une quelconque des revendications 1 à 5, **caractérisée par le fait qu'**elle contient un agent tensioactif cationique comme composant (B).

7. Composition d'article de soins capillaires décrite dans l'une quelconque des revendications 1 à 6, **caractérisée par le fait qu'**elle contient un agent tensioactif anionique et/ou un agent tensioactif amphotère comme composant (C).

8. Composition d'article de soins capillaires décrite dans l'une quelconque des revendications 1 à 7, **caractérisée par le fait qu'**elle contient un composé polymère pour la fixation des cheveux comme composant (D).
